# EUROPEAN PATENT APPLICATION

(11) **EP 1 702 579 A1**
(43) Date of publication of application: **20.09.2006**
(21) Application number: 05290556.9
(22) Date of filing: 14.03.2005
(51) Int. Cl.: A61B 19/00

(54) **Stabiliser with a direct support for the operator's hand**

(71) Applicant: INSTITUT NATIONAL DE LA SANTE ET DE LA RECHERCHE MEDICALE (INSERM), 75013 Paris (FR)
(72) Inventor: Miramand, Jean-Louis, 94800 Villejuif (FR); Paques, Michel, 75012 Paris (FR)
(74) Representative: Texier, Christian

(57) **Abstract**

Stabiliser (2,102,202,302), particularly for surgery, comprising a support for the operator's hand, the support (40) being arranged to form a fixed direct support for the hand in the partially closed position, in at least two perpendicular directions. The operating hand of a surgeon closes partially around the support (40). The support (40) is fixed in a certain position by means of a clamping mechanism (48). The support can be used in microsurgery, in particular in eye surgery.

## Description

The invention relates to stabilisers, particularly surgical stabilisers. It particularly relates to stabilisers for eye surgery.

The in situ administration of molecules in the human retina requires new developments in retinal microinjections. This has a variety of potential indications, either endovascular (treatment of central retinal vein occlusions, arterial occlusions, haemangioma embolisation) or intra or sub-retinal (administration of antiangiogenic substances, injection of stem cells or genic therapy vectors, etc.). However, improvements are required to be able to render such techniques usable in routine practice.

The main condition that seems likely to benefit from the development of intravascular microinjection is central retinal vein occlusion (CRVO). The pathogenesis of this disease is attributed to the presence of a thrombus in the intraneural segment of the central vein. In this disease, the use of thrombolytics has not given the expected results to date, possibly due to an availability problem at the occlusion site. Therefore, it is desirable to be able to perform intravascular administration.

The technical difficulties of intravascular retinal injection essentially lie in the constraints of the retinal approach, essentially the passage through the closed globe sclera, and the small size of retinal vessels (less than 125 microns). Intravenous retinal injection is performed on humans by only one centre in the world.

An aim of the invention is to propose a simple technique enabling constant manual control for satisfactory safety of the intravenous retinal injection procedure.

To this end, the invention provides for a stabiliser, particularly for surgery, comprising a support for the operator's hand, the support being arranged to form a fixed direct support for the hand in the partially closed position, in at least two perpendicular directions.

In addition, the stabiliser according to the invention may comprise at least any of the following characteristics:
- the support has a spherical shape;
- it comprises support position adjustment means with respect to a stabiliser base;
- it comprises an arm bearing the support and arm orientation adjustment means with respect to the support;
- it also comprises at least one second support for another part of the operator's arm;
- the second support is intended for the operator's forearm;
- the second support is intended for the operator's elbow;
- the second support comprises a tray;
- it comprises hand support position adjustment means with respect to the second support; and
- it comprises position adjustment means of the second support with respect to a stabiliser base.

Other characteristics and advantages of the invention will emerge in the following description of four embodiments given as non-limitative examples with reference to the appended figures wherein:
- figure 1 is a perspective view of a first embodiment of the stabiliser according to the invention;
- figures 2 and 3 are two left and top views, respectively of the stabiliser in figure 1;
- figure 4 is a larger-scale view of the end of the stabiliser in figure 1 bearing the ball;
- figures 5 and 6 are two views of an operator's hand using the stabiliser in figure 1 in two different positions;
- figure 7 and 8 are two respective perspective views of stabilisers according to the second and third embodiments of the invention; and
- figure 9 is a partial perspective view of a fourth embodiment of the stabiliser according to the invention.

In each of the four embodiments, the stabiliser is intended to be used for surgical purposes.

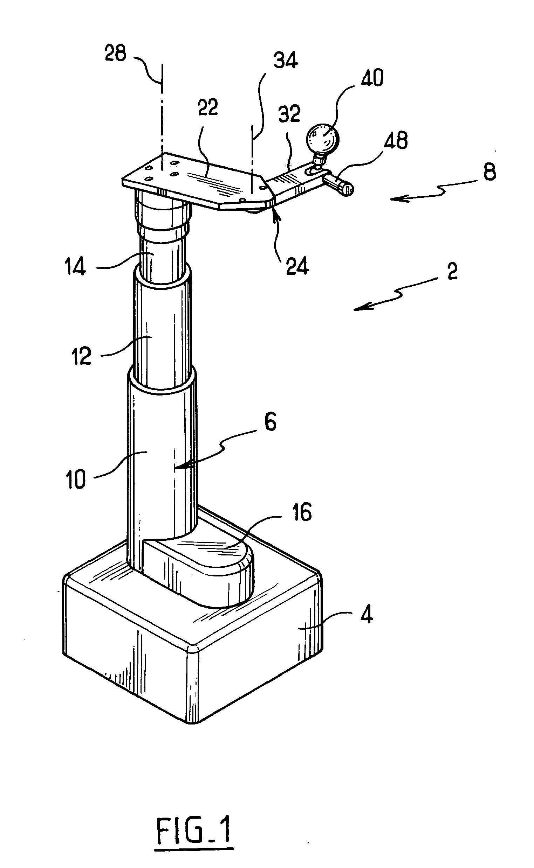

In the first embodiment illustrated in figures 1 to 6, the stabiliser 2 comprises a base 4, a telescopic column 6 and a support part 8.

In this instance, the base 4 is a box able to receive, if applicable, the stabiliser control electronics. In this case, it consists of a moulded aluminium box manufactured by OKATRON under the reference 4590130. This box has a rectangular parallelepiped shape as particularly seen in figure 1.

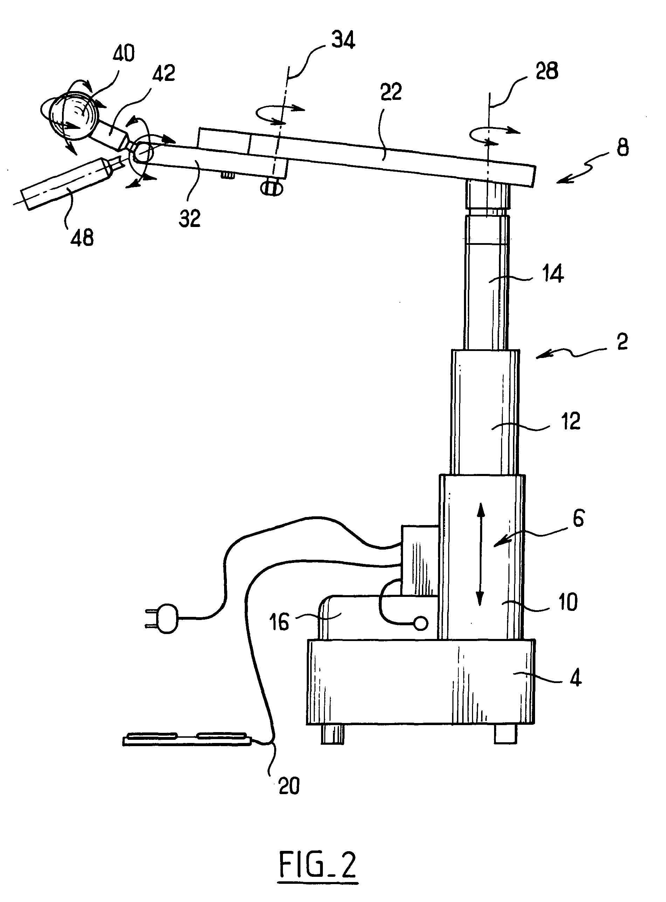

For example, the column 6 is a gamma column manufactured by DEWERT. In this instance, it comprises three cylindrical sections 10, 12 and 14, received coaxially in each other by being mobile with respect to each other sliding in the vertical direction. The lower end of the widest section 10 of the three is attached to the upper face of the box 4. This column is actuated by means of a worm screw and a motor housed in the motorisation compartment 16 adjacent to the column, via a reduction gear not shown. The column is powered, for example by the mains as illustrated in figure 2 and raised and lowered by the operator, for example via a pedal assembly 20.

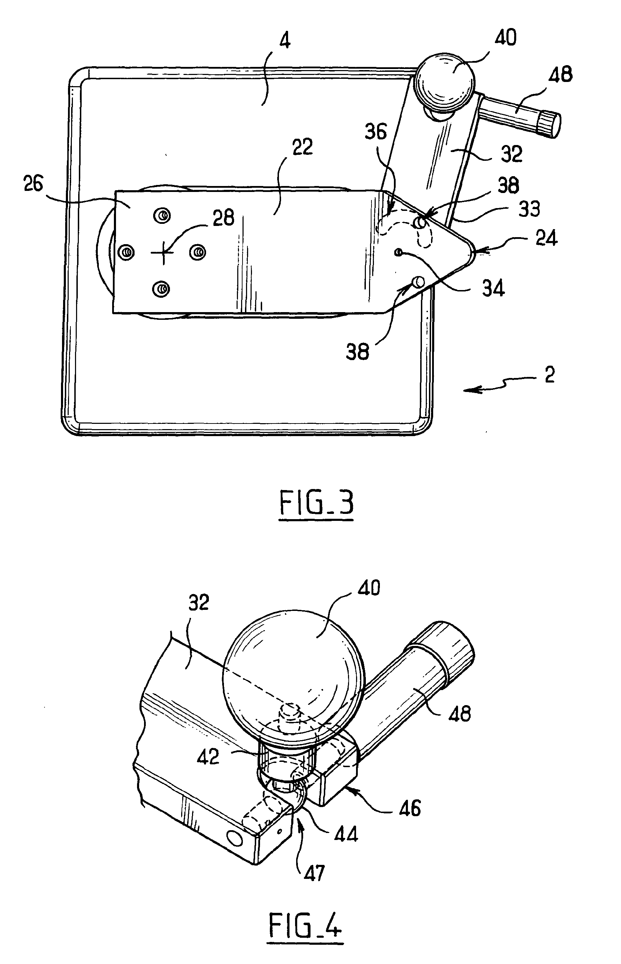

The support part 8 will now be described in more detail. Said part comprises a support in this case in the form of a flat tray 22 as illustrated in figure 2 and having in the plane view as illustrated in figure 4 a rectangular shape apart from the fact that its distal end is extended in the form of an isosceles triangle to show a rounded tip 24. The tray 22 has a proximal end 26 rigidly attached to the upper section 14 of the column. Alternatively, it may be envisaged for this attachment to be carried out using rotation assembly means making it possible to rotate the tray 22 around a vertical axis 28, for example combined with that of the column.

The support part 2 also comprises a second support in this case in the form of a block 32 of a general rectangular plane shape. A proximal end 33 of this block is attached to the distal end 24 of the tray 22. This attachment renders the block 32 mobile in rotation with respect to the tray 22 around a slightly inclined axis 34 with respect to the vertical direction. This slope stems from the fact that the tray 22 and the block 32 are parallel with each other while being slightly inclined with respect to the vertical direction shown by the axis 28, such that the distal end 24 of the tray 22 is slightly higher than its proximal end 26.

To enable the rotational mobility of the block 32 with respect to the tray 22, a suitable rotation shaft is inserted into two respective concentric orifices of these two parts. However, the block 32 can be locked in rotation with respect to the tray 22 in any position. For this, the block 32 comprises an arc-shaped groove drawn in dotted lines in figure 3, said groove 36 is liable to correspond with at least two orifices 38 provided on the tray 22. Therefore, it is simply necessary to insert, via the groove and one of the two orifices, a clamping device to lock the two parts together in any of an infinity of relative positions.

The support part 2 also comprises a hand support 40 formed in this instance by a ball having a spherical external face as a result. The ball 40 is connected to the block 32 by means of an arm 42 wherein an upper end is rigidly attached to the ball 40 and the other end, i.e. the lower proximal end 44, forms a ball joint with the block 32. For this, the latter is cut in its distal end 46 with a U-shaped opening 47 wherein the lower end 44 which has a spherical (convex) shape is housed. The latter is received between two shims in the form of portions of concave balls defined on the two opposite faces of the U-shaped housing, with radii identical to that of the ball 40. One of the shims is fixed while the other is mobile, forming the end of a clamping device 48 extending outside the block 32 and passing through half of its width to open into the U-shaped opening. The device 48 is connected with the block via a screw-nut joint. In this way, the end 44 forms a ball joint in the U-shaped opening with respect to the block 32, such that it is mobile with respect to the latter around multiple axes as indicated in figure 2. When the desired position is obtained, the operator can lock the ball 40 rigidly with respect to the block 32 by clamping the device 48 against the end 44.

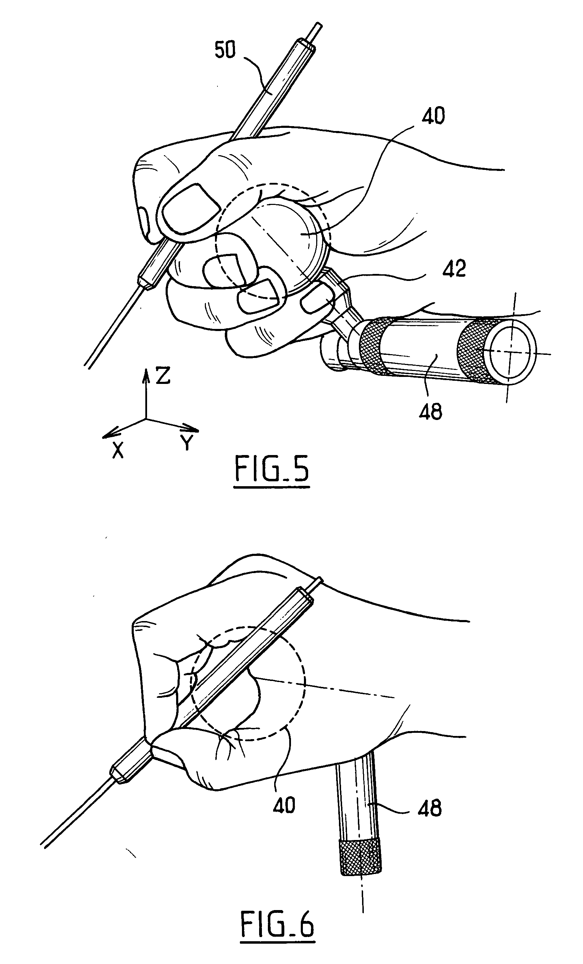

The tray 22 forms a support for the operator's elbow, at the end 26 and for its forearm along the tray 22. The wrist can rest on the tray 32. The ball 40 forms a support for the operator's hand when it is holding an elongated instrument such as that 50 illustrated in figure 5 and is thus in a partially closed position. To use the stabiliser, the operator closes his/her hand on the ball 40 while holding the instrument 50. As seen in figures 5 and 6, irrespective of the position of the hand on the ball, at least three or four of the fingers are in contact with the ball, or even the hand's five fingers, while ensuring a satisfactory grip of the tool in the hand. In figure 5, the hand is in a slightly pivoted position to show the inside of the hand with the ball from the operator's left. In figure 6, however, the back of the hand is facing upwards, such that this opening is facing downwards in this case. In particular, the support 40 forms a fixed support for the operator's hand in the three spatial directions X, Y and Z perpendicular with each other as illustrated in figure 5. As seen in figure 6, the adjustment device 48, which in this case is cylindrical in shape, extending laterally with respect to the ball and the block 32 can form an additional support for the base of the thumb or another part of the hand. The longitudinal axis of this device is horizontal.

Therefore, as can be seen, the microsurgical stabiliser consists of a passive manual external arm, wrist and hand stabiliser. All the degrees of freedom offered by the stabiliser enable the operator to select the position providing both optimum comfort and optimum efficiency for the operative procedure.

It can be observed that the middle finger of the operator's hand generally provides most of the stabilisation due to the fact it is around the ball on each of the three phalanxes. This stability is particularly advantageous if the tool 50 rests on this finger as illustrated in figure 5. Once the hand is immobilised in this way on the stabiliser and the cannula held by the surgeon has been inserted into the eye, the surgeon simply needs to make a micromovement to orient the cannula opposite the desired penetration site. In fact, the flexibility of the skin on the operator's hand and the glove generally covering this hand proves to sufficient to enable the micromovements required (over a range of less than 200 microns) of the fingers tips ensuring intravascular precision.

It may be envisaged to have several balls 40 available to be attached in a removable fashion to the arm 42 according to the surgeon's choice according to the size of the hand, the balls having different diameters from each other.

The support of the hand on the ball enables simple and comfortable adaptation to the morphology of the surgeon's hand without muscle contraction, while allowing, if required, an extension of the fingertips, thus enabling continuous manual control of the procedure by the surgeon. As can be seen, the invention does not require the use of a micro-manipulator since it enables passive stabilisation by means of extraocular support of the surgeon's hand.

In particular, it appears to be essential, in order to conduct the procedure, that the instrument is stable once it is in the injection vein, essentially laterally and in depth. The anteroposterior immobility is tolerable if the axis of the needle is coaxial with the vessels. In addition, rotation is easy to control manually.

The stabiliser makes it possible retain constant manual control of the instrument, which is important because, in the event of a movement of the patient or a preoperative incident, it is necessary to be able to remove it from the patient's body without delay.

Preferentially, the cannula used will be attached to a support consisting of the part of the tool 50 held by the surgeon.

It is reminded that since the technical difficulties of intravascular retinal injection essentially lie in the constraints of the retinal approach, essentially the passage through the closed globe sclera, and the small size of retinal vessels (less than 125 microns), the invention uses external stabilisation enabling constant manual control and wide distribution of the technique.

There are many other circumstances of use of the stabiliser for epi-, intra- or sub-retinal microinjection: in addition to branch venous occlusions, they consist of controlled sub-retinal injection of antiangiogenic substances for age-related degeneration, retinal haemangioma embolisation, Von Hippel Lindau's disease, the injection of stem cells or genic therapy vectors in cases of hereditary or acquired retinal dystrophy. More generally, the disease concerned may be retinal venous occlusions and retinal arterial occlusions.

It is observed that the stabiliser according to the invention enables the surgeon to operate on standard surgical tables or on his/her chair. The stabiliser also makes it possible to reduce the transmission of vibrations occurring at ground level to the surgeon's hand.

The eye surgery procedure may for example be that disclosed in the document US 6 402 734, apart from the fact that, in this case, it is only manual.

During a procedure, the following steps may be used:
- the subject to be operated on is positioned and prepared medically;
- the surgeon gets into position with his/her hand on the ball;
- the positioning of the device on the ground is adjusted;
- the height is adjusted by means of the column 6 of the device;
- the tray 22, block 32 and arm 42 are adjusted so that the instrument is at the same level as the eye;
- the clamping required to render the stabiliser completely rigid is performed;
- once the desired orientation has been obtained and the ball gripped in the user's hand, the instrument is lowered into the eye with microscope monitoring; and
- the movement of the hand with respect to the, ball makes it possible to determine and control the precise trajectory of the instrument in the eye, and the position of the injection.

It may be envisaged to remove the device 48 after performing the clamping required if the mobile shim clamping the end 44 of the arm 42 is independent from said device.

The shape of the support 40 may be a shape other than spherical. For example, it may be formed to fit the partially closed hand. It may have an ovoid or ellipsoid shape.

The ball 40 may be made of metal or, for example, from the synthetic material known as Delrin. The stabiliser may be manufactured so as to be fully autoclavable.

The shape of the tray 22 may also be modified to give it a non-plane shape, for example, such as a shell shape fitting the user's forearm.

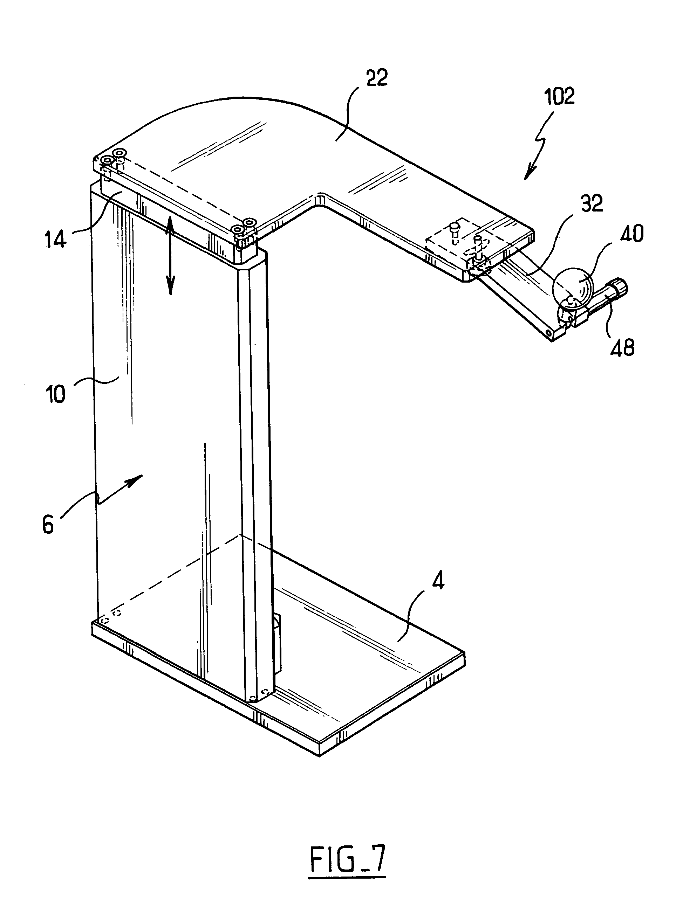

A second embodiment 102 of the stabiliser according to the invention is illustrated in figure 7.

In this case, the shape of the column 6 is flat and only comprises two mobile sections 10, 14 with respect to each other. It is arranged along one of the long sides of the base 4 which has a flat rectangular shape. The tray 22 is L-shaped, wherein one end is attached as above to the column and the other bears the block 32, itself supporting the ball 40. The tray 22 has a horizontal plane shape. As can be seen, in this example, the tray 32 does not have a plane shape but comprises two plane parts, sloping with respect to each other. The first part attached to the tray 22 is parallel to the latter while the other part emerging from the tray 22 slopes downwards.

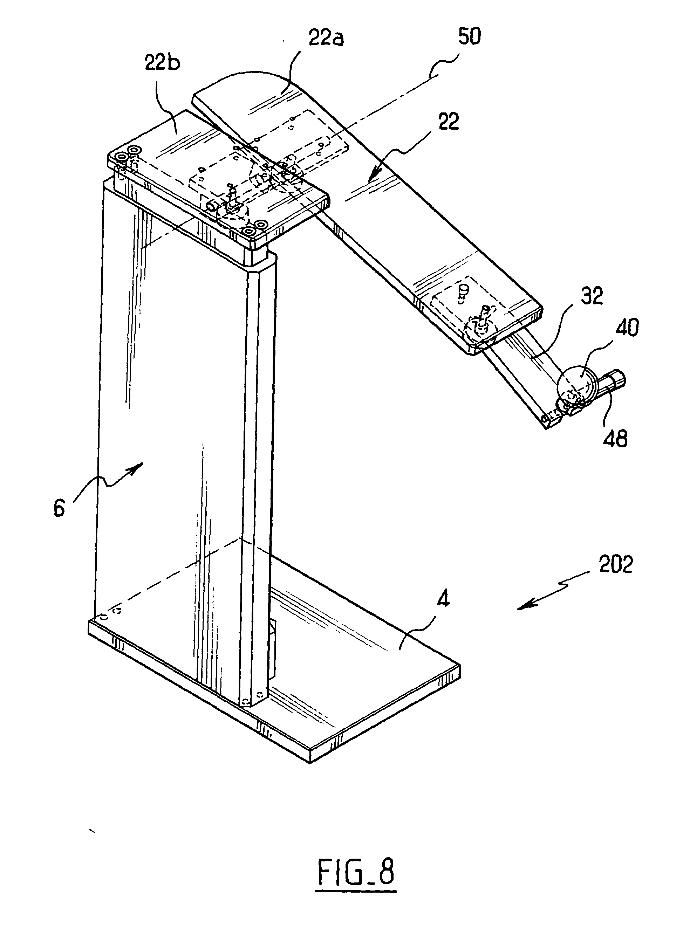

A third embodiment of the stabiliser 202 according to the invention is illustrated n figure 8. It is very similar to that in figure 7 in that the only modification consists of the tray 22 which comprises two parts 22a and 22b, mobile in rotation with respect to each other, around a horizontal axis 50 passing in this case through the thickness of the two parts 22a and 22b. This axis is perpendicular to the longitudinal direction of the part 22a of the tray bearing the block 32. Once the desired slope has been obtained by the surgeon from the choice of an infinity of positions, the two parts 22a and 22b can be locked with respect to each other by conventional clamping means. The rotation assembly means of these two parts with respect to each other are also conventional.

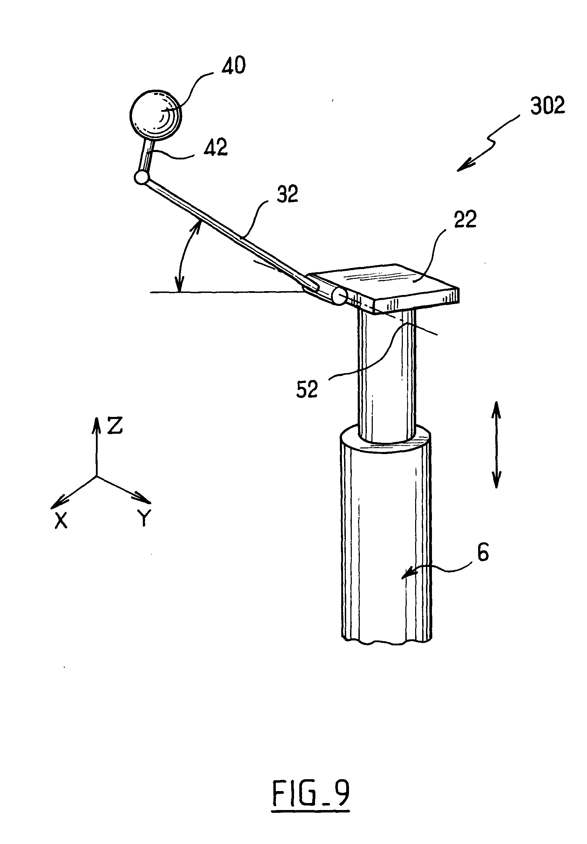

A fourth embodiment 302 is illustrated in figure 9.

The stabiliser comprises a base 4 and a column 6 identical to those in figure 1. The tray 22 is, in this case, shorter and essentially has a horizontal square shape centred on the column 6. An arm 32 replaces the block 32 and is mounted at its proximal end mobile in rotation with respect to the tray 22 around a horizontal axis 52 perpendicular to the longitudinal direction of the arm 32. At the distal end of the arm, the ball 40 is located with the ball joint associated with the arm 42.

This embodiment, which is preferred, is distinguished from those above in that it only offers two support points for the surgeon, i.e. the ball 40 for the hand and the tray 22 for the elbow, the arm being also entirely free, particularly at the forearm. It has in fact been observed that these two supports were sufficient to stabilise the entire hand, comfortably, while eliminating parasitic vibrations transmitted by the joints of the arm.

The invention is not restricted to the field of surgery.

It may be used by numerous operators who require a precise support to hold a tool. For example, it may be used in factories to help the operators who must repeat the same movement a lot of times, such as a movement having only 1 or 2 degrees of freedom. It may also be used for handywork, for example for painting on ceramics objects (vase, cups, plate, etc.).

It may also be used by disabled subjects as a movement assistance installation.

Naturally, numerous modifications may be made to the invention without leaving the scope thereof. The ball 40 may be truncated to consist only in a partial sphere.

## Claims

1. Stabiliser (2; 102; 202; 302), particularly for surgery, comprising a support for the operator's hand, **characterised in that** the support (40) is arranged to form a fixed direct support for the hand in the partially closed position, in at least two perpendicular directions (X, Y, Z).

2. Stabiliser according to the above claim, **characterised in that** the support (40) has a spherical shape.

3. Stabiliser according to any of the above claims, **characterised in that** it comprises support (40) position adjustment means (6, 22, 32, 42) with respect to a stabiliser base (4).

4. Stabiliser according to any of the above claims, **characterised in that** it comprises an arm (42) bearing the support (40) and arm orientation adjustment means (44, 48) with respect to the support.

5. Stabiliser according to any of the above claims, **characterised in that** it also comprises at least one second support (22, 32, 48) for another part of the operator's arm.

6. Stabiliser according to the above claim, **characterised in that** the second support (22) is intended for the operator's forearm.

7. Stabiliser according to any of claims 5 or 6, **characterised in that** the second support (22) is intended for the operator's elbow.

8. Stabiliser according to any of claims 5 to 7, **characterised in that** the second support (22) comprises a tray.

9. Stabiliser according to any of claims 5 to 8, **characterised in that** it comprises hand support (40) position adjustment means (32, 42) with respect to the second support (22).

10. Stabiliser according to any of claims 5 to 9, **characterised in that** it comprises position adjustment means (6) of the second support (22, 32, 42) with respect to a stabiliser base (4).
